# EUROPEAN PATENT SPECIFICATION

(11) **EP 3 159 029 B1**
(45) Date of publication and mention of the grant of the patent: **15.12.2021**
(21) Application number: 16002255.4
(22) Date of filing: 20.10.2016
(51) Int. Cl.: A61M 5/142

(54) **LIQUID INJECTOR FOR CONTINUOUSLY INJECTING FIXED QUANTITY OF CHARGED LIQUID**
FLÜSSIGKEITSINJEKTOR ZUR KONTINUIERLICHEN INJEKTION EINES FESTEN MENGE AN GELADENER FLÜSSIGKEIT
INJECTEUR DE LIQUIDE DESTINÉ À INJECTER EN CONTINU UNE QUANTITÉ FIXE DE LIQUIDE CHARGÉ

(30) Priority: 20.10.2015 KR 20150145929; 30.05.2016 KR 20160066369
(43) Date of publication of application: 26.04.2017
(73) Proprietor: Ace Medical Co., Ltd, Gyeonggi-Do 412-520 (KR)
(72) Inventor: LEE, Jong-Woo, 136-060 Seoul (KR)
(74) Representative: Hering, Hartmut

(56) References cited:
- EP-A2- 0 941 741
- EP-A2- 2 233 166
- WO-A1-01/97876
- US-A1- 2012 157 918
- US-A1- 2015 105 725
- None

## Description

### TECHNICAL FIELD

The present disclosure relates to a liquid injector that additionally supplies charged liquid to a patient. More particularly, the present disclosure relates to a liquid injector that continuously supplies a fixed quantity of liquid to a patient only when the fixed quantity of liquid has been charged.

### BACKGROUND

In general, if a patient has a surgical operation, he or she feels a pain around where he or she has had the surgery. Then, the degrees of pain felt by the patients are different according to the kinds of the surgical operations and operation spots, and in particular, the degrees of pain felt by the patients are different, depending on the individual characteristics of the patients even though the same kind of surgical operations are conducted.

Accordingly, together with a liquid injector (KR 10-0519247 B1) that continuously injects an analgesic to alleviate the pain of the patient, a liquid injector patient controlled analgesia (PCA) that can additionally inject an analgesic when the patient complains of pain are necessary. That is, a liquid injector is installed between a liquid supply and a catheter according to the related art, and catches and charges the liquid at an intermediate point when the liquid supply continuously sends the liquid to the catheter and additionally sends the liquid whenever it is necessary to alleviate the pain of the patient.

However, the catheter connected to the liquid injector is inserted into a vein or an epidural space of a patient to inject liquid into the body of the patient, and the catheter generally has a diameter of about 2 mm and is thin and long and thus the quantity of injected liquid is small and the injection speed of the liquid is also low.

Accordingly, the recharging time of the liquid injector for catching and charging the liquid at an intermediate point is long because the injection speed of the liquid is low, and in particular, even when the charged liquid is to be additionally introduced by pushing the button of the liquid injector, the quantity of the introduced liquid is not constant so that it is difficult to identify how much the liquid has been injected into the body of the patient.

That is, as the liquid is repeatedly injected into the body of the patient while the liquid is not completely charged, it is not identified how much the liquid corresponding to a narcotic analgesic is injected into the body of the patient.

The above information is presented as background information only to assist with an understanding of the present disclosure. No determination has been made, and no assertion is made, as to whether any of the above might be applicable as prior art with regard to the present disclosure.

EP 2 233 166 A2, EP 0 941 741 A2, US 2012/157918 A1 and WO 01/97876 A1, respectively, disclose a liquid injector comprising a fixed quantity charging unit, a push button, a piston, a brake button unit, the push button is released, a continuous injection unit and a resilient body.

US 2015/105725 A1, which document is considered as being the most relevant prior art document, discloses a liquid injector according to the preamble of claim 1.

The problem to be solved by the present invention is regarded as how to provide a liquid injector that can inject a fixed quantity of liquid into the body of a patient while automatically charging the liquid and can continuously supply the fixed quantity of liquid into the body of the patient so that the liquid injector can be safely used.

### SUMMARY

Aspects of the present disclosure are to address at least the above-mentioned problems and/or disadvantages and to provide at least the advantages described below. Accordingly, an aspect of the present disclosure is to provide a liquid injector that can inject a fixed quantity of liquid into the body of a patient while automatically charging the liquid and can continuously supply the fixed quantity of liquid into the body of the patient so that the liquid injector can be safely used.

In accordance with an aspect of the present disclosure, a liquid injector that additionally supplies charged liquid while continuously injecting a small quantity of liquid is provided. Especially, the liquid injector includes a fixed quantity charging unit, a brake button unit, and a continuous injection unit as described in detail by the features of claim 1. Further advantageous developments of the present invention are described in dependent claims 2 to 7.

The liquid injector according to the present disclosure can charge a fixed quantity of liquid while automatically charging the liquid, and can continuously inject the liquid into the body of the patient little by little while absorbing the fixed quantity of discharged liquid so that the liquid injector can be safely used.

Other aspects, advantages, and salient features of the disclosure will become apparent to those skilled in the art from the following detailed description, which, taken in conjunction with the annexed drawings, discloses various embodiments of the present disclosure.

### BRIEF DESCRIPTION OF THE DRAWINGS

The above and other aspects, features, and advantages of certain embodiments of the present disclosure will be more apparent from the following description taken in conjunction with the accompanying drawings, in which:
FIG. 1 is a view illustrating an in-use state of a liquid injector according to an embodiment of the present disclosure;
FIG. 2 is a perspective view illustrating the liquid injector according to an embodiment of the present disclosure;
FIG. 3 is an exploded perspective view illustrating the liquid injector according to an embodiment of the present disclosure;
FIG. 4 is a view illustrating an operational state of the liquid injector according to an embodiment of the present disclosure;
FIG. 5 is a perspective view illustrating a brake button unit of the liquid injector according to an embodiment of the present disclosure;
FIG. 6 is an exploded perspective view illustrating the brake button unit of the liquid injector according to an embodiment of the present disclosure;
FIG. 7 is a view illustrating an operational state of the brake button unit of the liquid injector according to an embodiment of the present disclosure;
FIGS. 8 and 9 are an operational state view and an exploded perspective view of a brake button unit of the liquid injector according to another embodiment of the present disclosure;
FIGS. 10 and 10A are a perspective view and a sectional view illustrating a state in which a key of the liquid injector according to the embodiment of the present disclosure is mounted;
FIG. 11 is a partial perspective view of the liquid injector according to the embodiment of the present disclosure;
FIG. 12 is a perspective view illustrating a continuous injection unit of the liquid injector according to an embodiment of the present disclosure;
FIG. 13 is an exploded perspective view illustrating the continuous injection unit of the liquid injector according to an embodiment of the present disclosure; and
FIG. 14 is a view illustrating an operational state of the continuous injection unit of the liquid injector according to an embodiment of the present disclosure.

Throughout the drawings, it should be noted that like reference numbers are used to depict the same or similar elements, features, and structures.

### DETAILED DESCRIPTION

The following description with reference to the accompanying drawings is provided to assist in a comprehensive understanding of various embodiments of the present disclosure as defined by the claims and their equivalents. It includes various specific details to assist in that understanding but these are to be regarded as merely exemplary. Accordingly, those of ordinary skill in the art will recognize that various changes and modifications of the various embodiments described herein can be made without departing from the scope of the present disclosure. In addition, descriptions of well-known functions and constructions may be omitted for clarity and conciseness

The terms and words used in the following description and claims are not limited to the bibliographical meanings, but, are merely used by the inventor to enable a clear and consistent understanding of the present disclosure. Accordingly, it should be apparent to those skilled in the art that the following description of various embodiments of the present disclosure is provided for illustration purpose only and not for the purpose of limiting the present disclosure as defined by the appended claims and their equivalents.

It is to be understood that the singular forms "a," "an," and "the" include plural referents unless the context clearly dictates otherwise. Thus, for example, reference to "a component surface" includes reference to one or more of such surfaces.

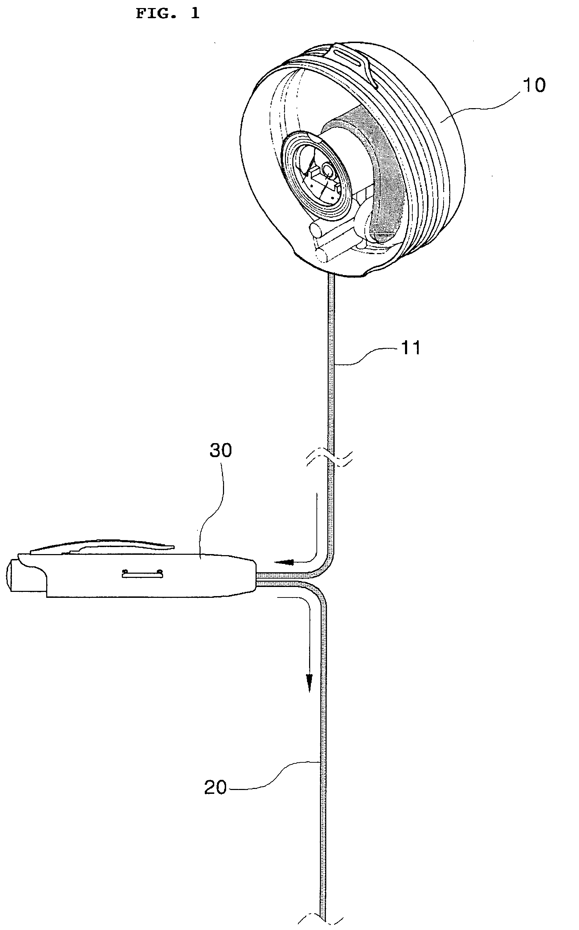

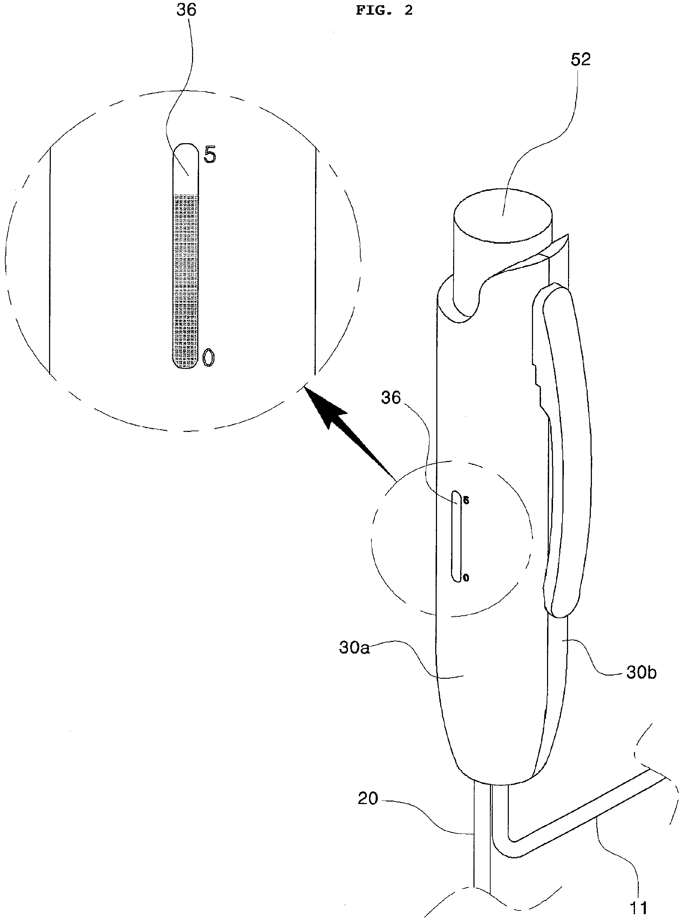

FIG. 1 illustrates a connection state of a liquid injector, a liquid supply, and an injection hose 20 according to an embodiment of the present disclosure. FIG. 2 is a perspective view illustrating the liquid injector according to an embodiment of the present disclosure.

Referring to FIGS. 1 to 2, as illustrated, the present disclosure includes a liquid supply 10 that accommodates a liquid, an injection hose 20 that injects the liquid sent from the liquid supply 10 into the body of the patient, and a liquid injector 30 that partially catches and charges the liquid that is being sent to the injection hose 20 at an intermediate point when the liquid supply 10 supplies the liquid to the injection hose 20.

Then, the liquid injector 30 extends from one side to another side, and a pair of cases 30a and 30b are assembled such that an inlet port 31 connected to a hose **11** of the liquid supply 10, together with an outlet port 32 connected to the injector hose 20 are formed at one side thereof and a push button 52 is installed at an opposite end thereof so that if the push button 52 is pushed, the liquid charged in the liquid injector 30 is further supplied to the patient in addition to the liquid that is being normally supplied by the liquid supply 10.

Then, because a transparent window 36 is formed on one side of the case of the liquid injector 30 and a cylinder 40 in the case is also formed of a transparent material, a liquid can be visually identified through the transparent window if the cylinder is filled with the liquid.

Then, the liquid injector 30 includes a fixed quantity charging unit 43 that charges a fixed quantity of liquid, a brake button unit 53 that discharges the liquid charged in the fixed quantity charging unit 43, and a continuous injection unit 64 that continuously sends the liquid to the injector hose 20 little by little while accommodating the liquid discharged by the brake button unit 53 so that the liquid is discharged only when a fixed quantity of liquid is charged in the fixed quantity charging unit 43 and then the discharged liquid is continuously injected into the body of the patient little by little.

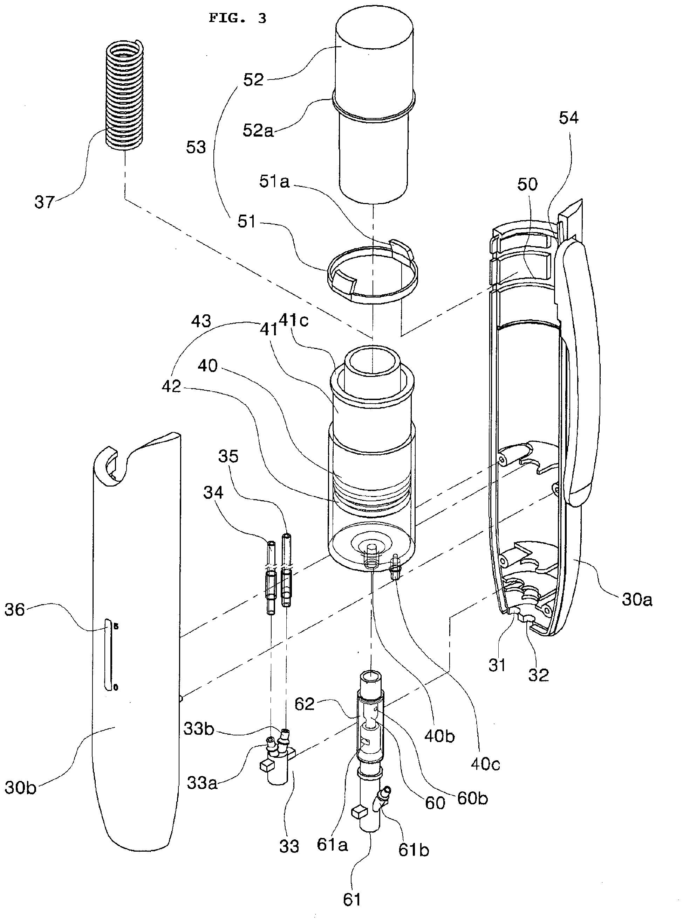

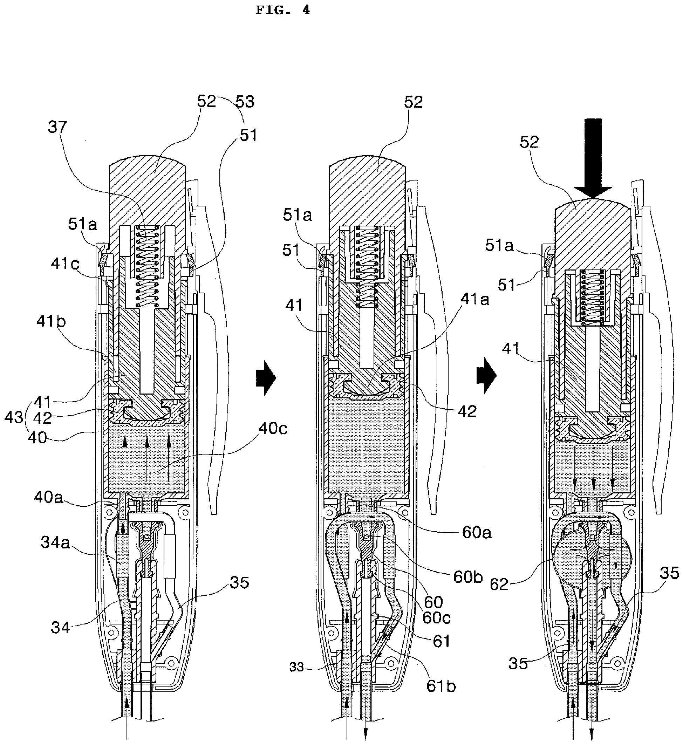

For reference, FIG. 3 is an exploded perspective view of the liquid injector according to the embodiment of the present invention. FIG. 4 illustrates a process of charging and discharging liquid to and from the liquid injector according to an embodiment of the present disclosure.

Referring to FIGS. 3 to 4, a Y-shaped branch pipe 33 and a connection pipe 61 are installed in the inlet port 31 and the outlet port 32 formed at a tip end of the case, one side pipe 33a of the branch pipe 33 is connected to a charging hole 40a of a cylinder 40 by a connection hose 34, and a check filter 34a is installed between the charging hole 40a and the branch pipe 33 such that the liquid flows only in one direction.

The connection pipe 61 of the inlet port 31 is connected to a connector 60 of the cylinder 40 by using a resilient tube 62 and a protruding pipe 61b of the connection pipe 61 is connected to an opposite side pipe 33b of the branch pipe 33 with another connection hose 35 so that if the liquid is normally introduced inwards through the inlet port 31 such that a fixed quantity of liquid is charged in the cylinder 40, the entire quantity of liquid introduced through the inlet port 31 flows to the connection pipe 61 through the branch pipe 33 and is introduced into the body of the patient.

If the liquid is not charged in the cylinder 40, a partial or entire quantity of the liquid introduced through the inlet port 31 flows to the cylinder 40 and is charged in an inner accommodation space 40c. Then, the piston 41 starts to be lifted by the liquid introduced into the cylinder 40. Moreover, if the piston 41 is lifted to a predetermined height, a fixed quantity of liquid is charged.

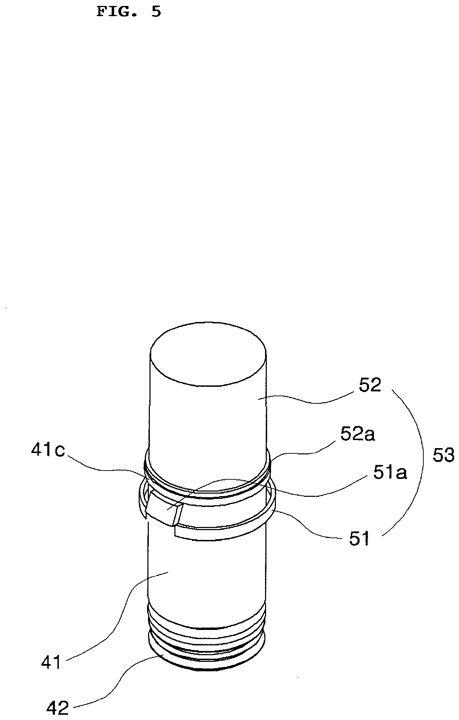

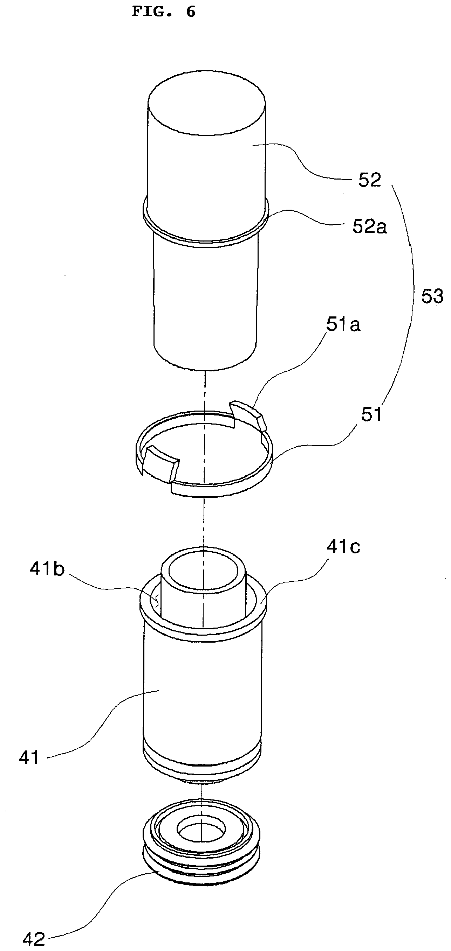

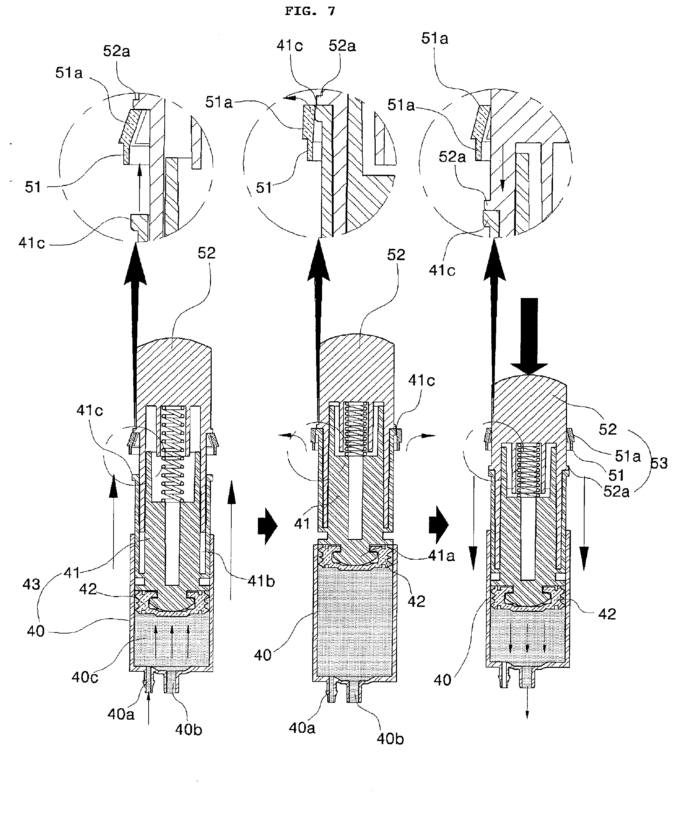

FIG. 5 is a perspective view illustrating the brake button unit and the piston according to an embodiment of the present disclosure. FIG. 6 is an exploded perspective view of FIG. 5 according to an embodiment of the present disclosure. FIG. 7 is a flowchart illustrating an operation of the piston and the brake button unit according to an embodiment of the present disclosure.

Referring to FIGS. 5 to 7, the fixed quantity charging unit 43 charges a fixed quantity of liquid in the interior of the cylinder 40 when the piston 41 is lifted to a lower end of the push button 52 installed at an upper end of the case, is raised to a predetermined height while being adhered to the lower end of the push button 52, and is stopped.

Then, if the push button 52 adhered to the piston 41 is pushed, the fixed quantity of liquid charged in the interior of the cylinder 40 is discharged.

For reference, a boss 41a is formed at a lower end of the piston 41, a packing 42 is mounted on the boss 41a and is adhered to an inner wall of the cylinder 40, an insertion space 41b is formed at an upper portion of the piston 41, and a lower end of the push button 52 is inserted into the insertion space 41b. Then, a protruding step 41c is formed at an upper periphery of the piston 41.

A stop groove 50 is formed in the cases 30a and 30b in which the piston 41 and the cylinder 40 are installed, and an annular stop rim 51 having a ring shape is positioned in the stop groove 50.

A pair of stop pieces 51a protrude from an upper portion of the stop rim 51 to be inclined inwards, and the piston 41 is inserted into the stop rim 51.

Accordingly, if the piston 41 is lifted by the liquid charged in the interior of the cylinder 40, the protruding step 41c of the piston 41 pushes out the stop pieces 51a of the stop rim 51 located on the outer side so that the stop pieces 51a are opened while being resiliently supported.

The push button 52 having a support step 52a at a lower end thereof is installed at an upper portion of the piston 41, a lower portion of the push button 52 is inserted into the insertion space 41b of the piston 41, and the support step 52a formed at an outer side of the push button 52 is supported by the stop piece 51a and is stopped. Then, a resilient body is inserted into the insertion space 41b to return the push button 52 lowered downwards to an original state.

Accordingly, when the fixed quantity charging unit 43 is fully filled with the liquid and the piston 41 is adhered to the push button 52, the protruding step 41c formed on the outer side of the piston 41 resiliently supports and opens the stop pieces 51a and is adhered to the support step of the push button 52.

Accordingly, when a predetermined quantity of liquid is not charged in the fixed quantity charging unit 43, the support step 52a of the push button 52 is supported by the stop pieces 51a and the downward movement of the push button is restricted, but if a predetermined quantity of liquid is charged in the fixed quantity charging unit 43 such that the protruding step 41c of the piston 41 resiliently supports and opens the stop pieces 51a, the protruding step 41c and the support step 52a of the push button 52 are adhered to each other. Then, because the stop pieces are opened if the push button is pushed, the support step and the protruding step are lowered along a guide rod 54 while being adhered to each other so that the fixed quantity of liquid accommodated in the interior of the cylinder is discharged.

For reference, according to the embodiment of the present disclosure, the stop rim 51 may be widened or closed while being easily resiliently supported by the piston by cutting away a lower portion of the stop pieces 51a.

If a fixed quantity of liquid charged by the fixed quantity charging unit 43 and the brake button unit 53 is discharged, the liquid is sent to the outside of the cylinder 40 though a discharge hole 40b.

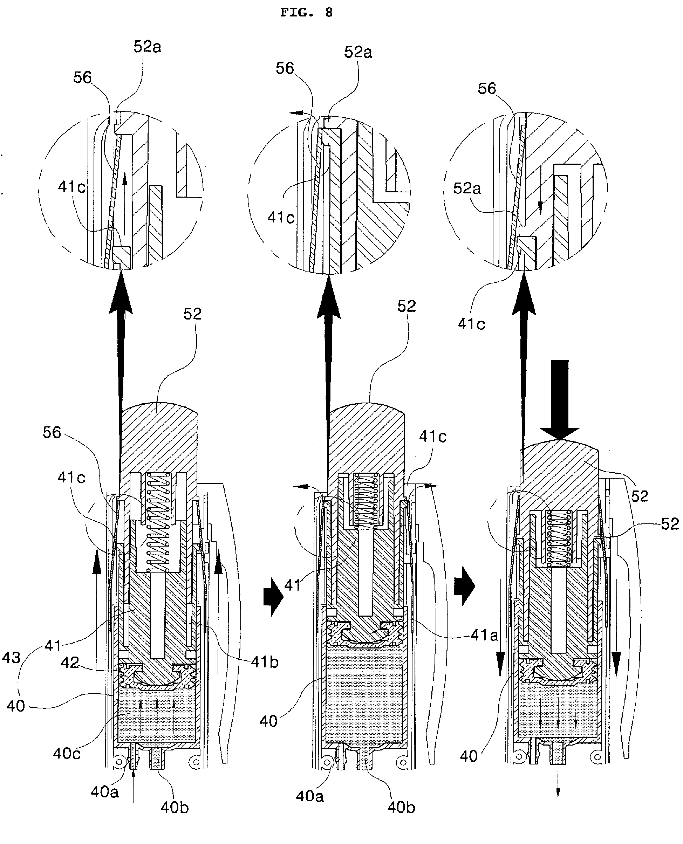

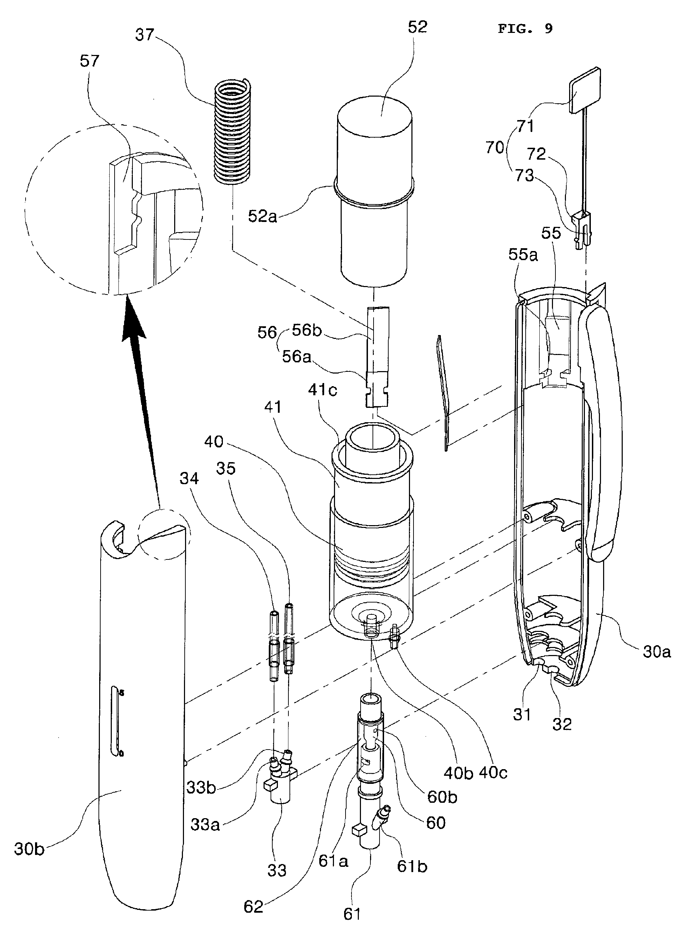

For reference, FIG. 8 illustrates a brake button unit according to an embodiment of the present invention. FIG. 9 illustrates a state in which the liquid injector of FIG. 8 is exploded.

Referring to FIGS. 8 to 9, a boss 41a is formed at a lower end of the piston 41, a packing 42 is mounted on the boss 41a and is adhered to an inner wall of the cylinder 40, an insertion space 41b is formed at an upper portion of the piston 41, and a lower end of the push button 52 is inserted into the insertion space 41b. Then, a protruding step 41c is formed at an upper periphery of the piston 41.

A stop g groove 55 having a fixing boss 55a is formed on the inside of the case 30a and 30b, the stop g groove 55 extends long up to a lower portion of an upper end periphery of the case, on which the push button 52 is mounted, a lock spring 56 having a recess 56a corresponding to the fixing boss 55a at one side thereof and an inwardly inclined piece 55b at an opposite side thereof is mounted on the stop g groove 55.

Then, one side of the lock spring 56 is fixed by the fixing boss 55a but the inclined piece 55b on the opposite side may be moved, and an end of the inclined piece 55b is located between the support step 52a of the push button 52 and the protruding step 41c of the piston.

Accordingly, the push button 52 is stopped by the inclined piece 55b of the lock spring 56 such that a downward movement of the push button 52 is braked in a normal state, and the piston 41 is moved downwards together with the push button 52 so that a fixed amount of liquid filled in the cylinder 40 may be discharged to the outside only when a fixed amount of liquid is filled in the interior of the cylinder as the piston 41 ascends to the end such that the inclined piece of the lock spring 56 is pushed to the outside by the protruding step 41c and the support end 52a of the push button 52 and the protruding step 41c of the piston make contact with each other.

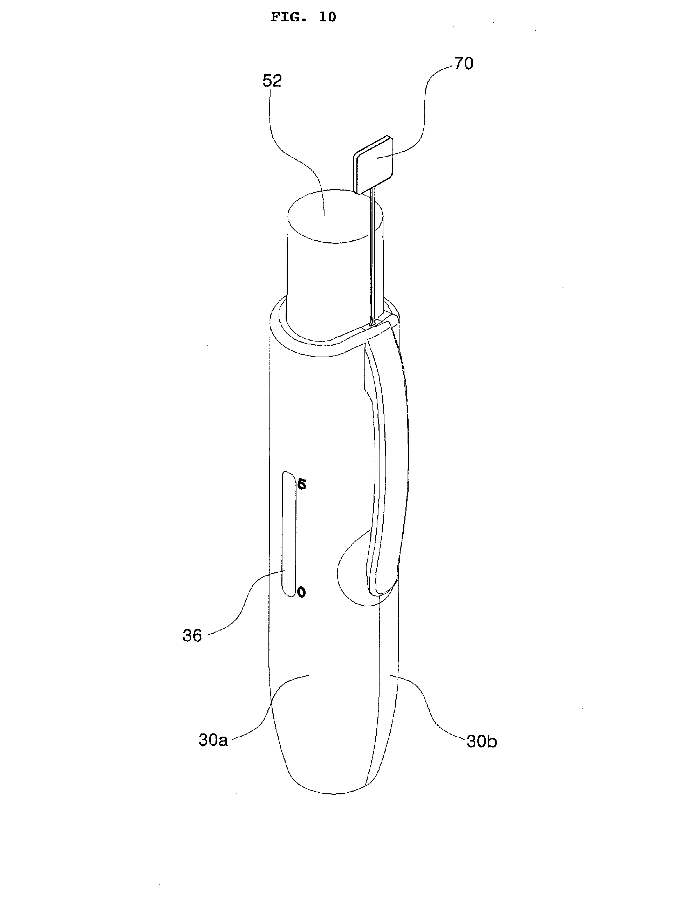

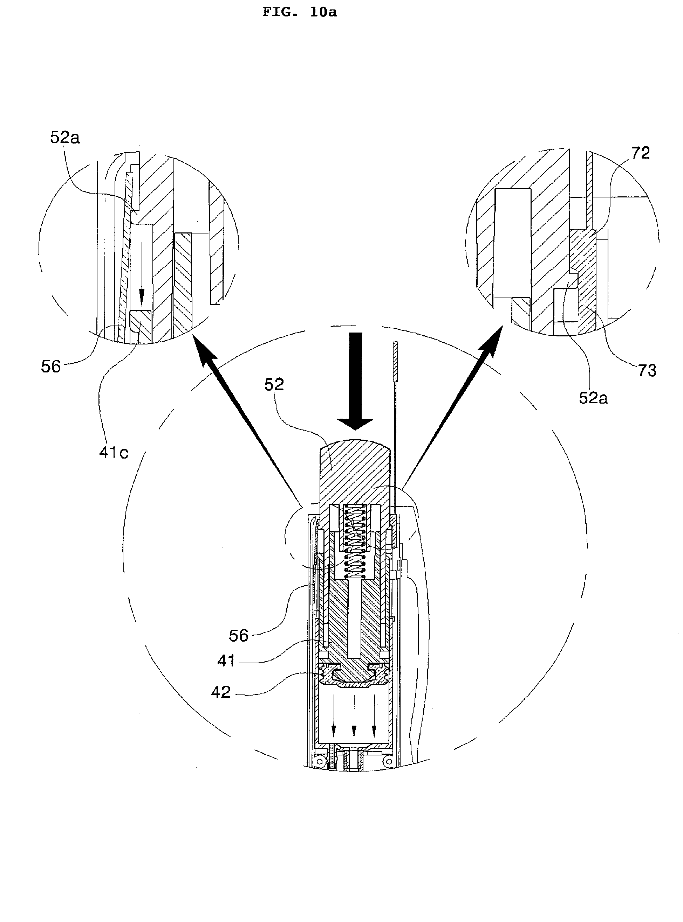

FIGS. 10 and 10A illustrate a state in which a key is mounted on an upper end of the case such that air in the cylinder may be easily deflated during an initial stage of use.

Referring to FIGS. 10 to 10A, a mounting recess 57 is formed inside an upper end of the case 30a and 30b, and a key 70 that allows the support step 52a of the push button 52 to be located inside the stop piece 51a or the inclined piece 56b of the lock spring 56 is detachably mounted on the mounting recess 57.

Then, the key 70 includes a knob 71 protruding to the upper side of the push button, a mounting boss 72 that allows the support step 52a of the push button 52 to be located inside the stop piece 51a or the inclined piece 56b of the lock spring 56 when being inserted into the mounting recess, and a pair of resilient support pieces formed at a lower portion of the mounting boss 72.

Further, if the key 70 is inserted into the mounting recess 57 between the push button 52 and the case 30a and 30b, the resilient support piece is inserted into and fixed to the mounting recess while being resiliently supported, and the mounting boss 72 prevents the support step 52a of the push button 52 or the inclined piece 56b of the lock spring 56 from being moved to the upper side of the stop piece 51a of the stop rim 51 so that the push button 52 is freely pushed and thus the air in the cylinder in the initial stage of use is easily removed.

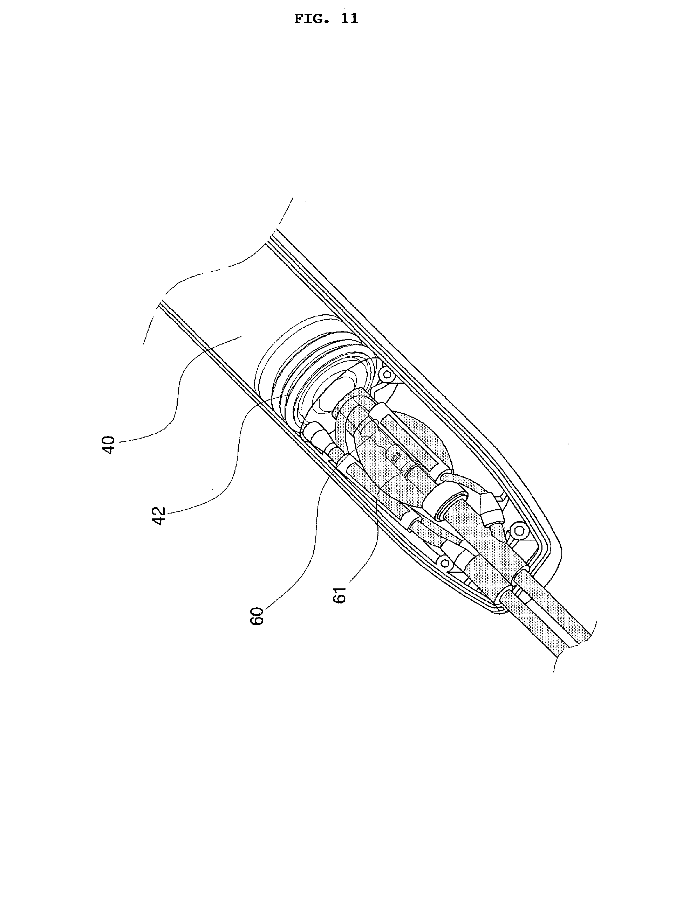

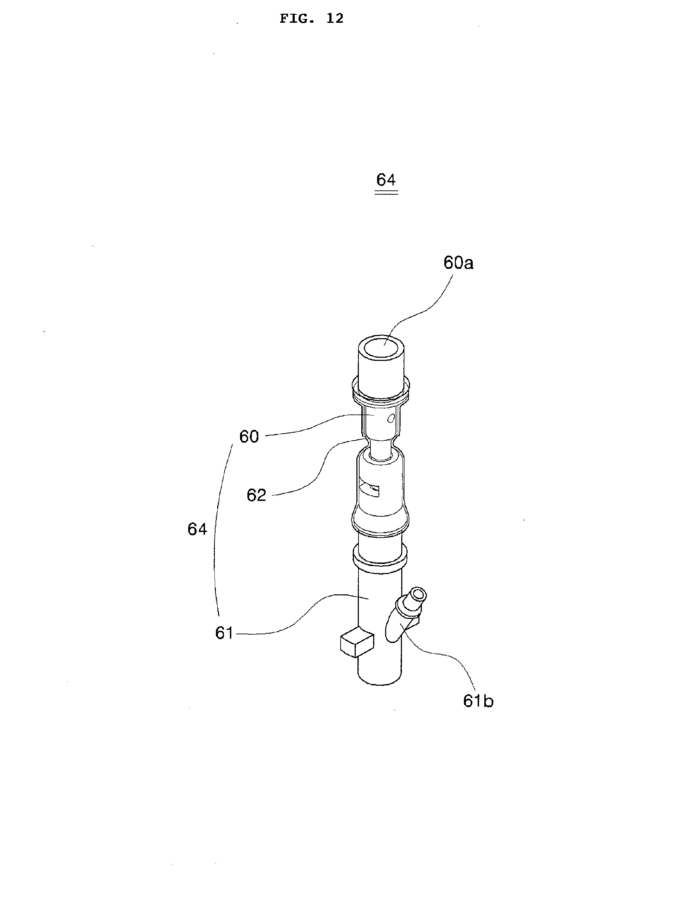

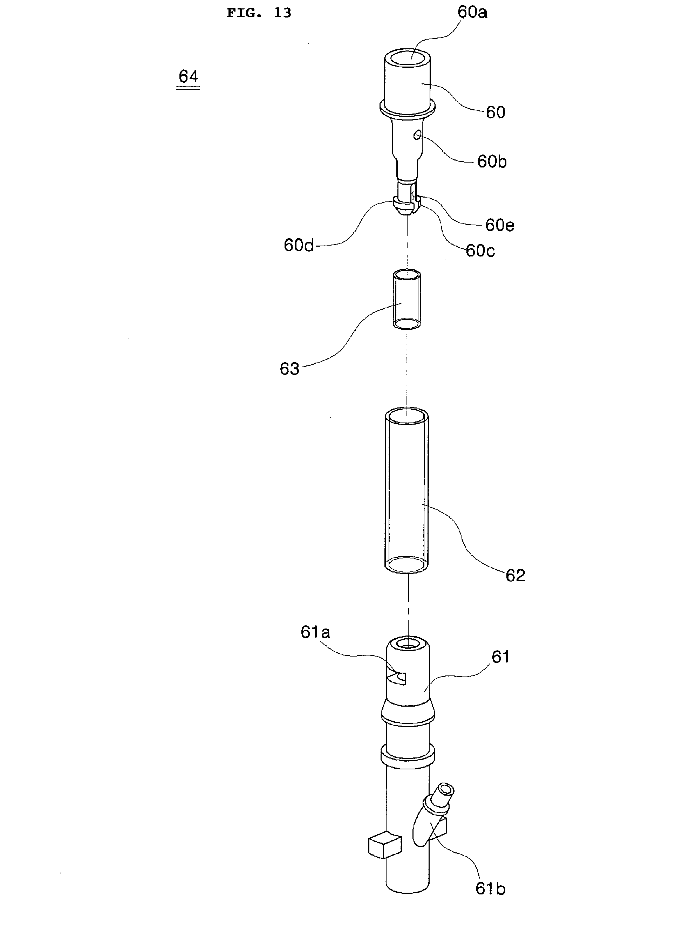

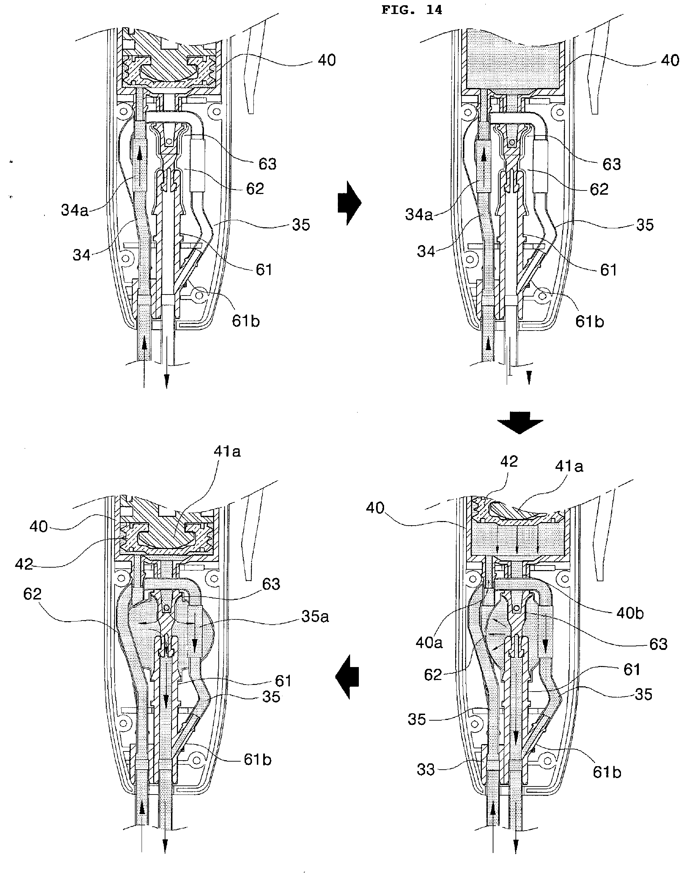

FIG. 11 is a partial perspective view of the liquid injector according to the embodiment of the present disclosure. FIG. 12 illustrates the continuous injection unit 64 mounted between the discharge hole 40b and the outlet port 32 according to an embodiment of the present disclosure. FIG. 13 illustrates a disassembled state of the continuous injection unit 64 according to an embodiment of the present disclosure. FIG. 14 is a flowchart illustrating an operation of the continuous injection unit according to an embodiment of the present disclosure.

Referring to FIGS. 11 to 14, the continuous injection unit 64 accommodates a fixed quantity of liquid while a resilient tube 62 is expanded.

Then, the liquid absorbed in the resilient tube 62 is continuously sent to the outside of the outlet port 32 by a contraction force of the resilient tube 62 that returns the resilient tuber 62 to an original state and is injected into the body of the patient.

Then, one side of the connector 60 is inserted into the discharge hole 40b of the cylinder 40 and is fixed, an opposite side of the connector 60 is inserted into the connection pipe 61 and is fixed, an inward recess 60a is formed at one side of the connector 60, and an injection hole that laterally passes through the inward recess 60a is formed in the inward recess 60a. One end of the resilient tube 62 is fixed to the upper side of the injection hole.

Accordingly, the fixed quantity of liquid discharged to the outside of the discharge hole 40b of the cylinder 40 is accommodated in the interior of the resilient tube 62 through an inward hole 60b of the connector 60, and then the resilient tube 62 absorbs the fixed quantity of liquid discharged to the outside of the cylinder 40 while being expanded.

Then, an opposite end of the connector 60 is cut away longitudinally with respect to the center thereof to form a pair of coupling pieces 60c and coupling steps 60d are formed at ends of the coupling pieces 60c so that when the coupling pieces 60c are inserted into the connection pipe 61, the coupling steps 60d are located inside coupling recesses 61a of the connection pipe 61 and are stopped.

Accordingly, because the resilient tube 62 mounted on the outside is expanded and contracted only in one direction with respect to the coupling pieces if the connector 60 and the connection pipe 61 are connected to each other, the liquid is accommodated in the resilient tube while the resilient tube is neither deflected nor bent as the restoring force of the resilient tube is maximized. Furthermore, as the liquid accommodated in the resilient tube 62 is introduced into the connection pipe 61 through a cutaway portion 60e between the coupling pieces 60c by a restoring force of the resilient tube 62, the fixed quantity of liquid is continuously sent to the outside of the outlet port 32 little by little.

Then, the resilient tube 62 may accommodate liquid, a quantity of which is larger than the quantity of the liquid charged in the cylinder 40, but only one dose of liquid is accommodated by making the space, in which the resilient tube 62 is expanded, the same as the area of the cylinder 40.

Moreover, according to the embodiment of the present disclosure, a fixed tube 63 that is resilient may be installed inside the resilient tube 62. That is, the fixed tube 63 is installed while surrounding the injection hole of the connector 60 and the resilient tube 62 is fixedly installed on the fixed tube 63 so that if the liquid accommodated in the interior of the cylinder 40 is discharged, the liquid pushed to the outside of the injection hole is accommodated inside the resilient tube 62. Furthermore, if no liquid is pushed to the outside of the injection hole, the fixed tube is contracted to block the injection hole.

Furthermore, when the expanded resilient tube 62 pushes out the liquid, the fixed tube 63 prevents the liquid from returning into the injection hole so that only the resilient tube located at an intermediate point absorbs the liquid and then sends the liquid to the outside.

According to the present disclosure, one dose of liquid is discharged only when the charging is completed if the push button is pushed, and then the discharged liquid is absorbed in the resilient tube 62 and then a small quantity of liquid is continuously sent to the body of the patient by a returning force to be safely used.

While the present disclosure has been shown and described with reference to various embodiments thereof, it will be understood by those skilled in the art that various changes in form and details may be made therein without departing from the scope of the present disclosure as defined by the appended claims and their equivalents.

## Claims

1. A liquid injector (30) that additionally supplies charged liquid while continuously injecting a small quantity of liquid, the liquid injector (30) comprising:
a fixed quantity charging unit (43) that charges a fixed quantity of liquid, a brake button unit (53) that discharges the liquid charged in the fixed quantity charging unit (43), and a continuous injection unit (64) that continuously sends the liquid to an injector hose (20) little by little while accommodating the liquid discharged by the brake button unit (53) so that the liquid is discharged only when a fixed quantity of liquid is charged in the fixed quantity charging unit (43) and then the discharged liquid is continuously injected into the body of the patient little by little, wherein
said fixed quantity charging unit (43) is adhered to a lower end of a push button (52) when a piston is lifted by liquid introduced into the interior of a cylinder and then is stopped to charge a fixed quantity of liquid in the interior of the cylinder;
said brake button unit (53), if the lifted piston and the push button (52) are adhered to each other while the downward movement of the push button (52) is stopped, lowers the push button (52) together with the piston downwards as the stopped downward movement of the push button (52) is released; and
said continuous injection unit (64), by a restoring force of a resilient tube (62) that is expanded while accommodating a fixed quantity of liquid discharged to the outside of the cylinder by the brake button unit (53), continuously pushes out the liquid accommodated in the interior of the resilient tube (62),
**characterized in that**
said fixed quantity charging unit (43) comprises:
a cylinder (40) that has a charging hole and a discharge hole at one side thereof and that has an accommodation space at an opposite side thereof;
a piston (41) a lower end of which is inserted into the accommodation space of the cylinder (40), which has an insertion space (41b) at an end thereof, and which has a protruding step (41c) at a periphery thereof;
a resilient body that is inserted into the insertion space (41b) of the piston (41); and
a push button (52) that has a support step (52a) at a periphery thereof above the resilient body, and
wherein the protruding step (41c) and the support step (52a) of the push button (52) are adhered to each other when a piston (41) is lifted by the liquid introduced into the interior of the cylinder (40), and then is stopped at a predetermined location to charge a fixed quantity of liquid in the interior of the cylinder (40);
said brake button unit (53) comprises:
a ring-shaped stop rim (51) that has inward stop pieces (51a); and
cases (30a, 30b) each of which has a stop groove (50) in which the stop rim (51) is positioned and has a guide rod (54),
wherein the piston (41) is mounted on the inside of the cases (30a, 30b) while being inserted into the stop rim (51), and
wherein, if the piston (41) is lifted, a support step (52a) of the push button (52) mounted on the stop pieces (51a) and the protruding step (41c) of the piston (41) are adhered to each other while the stop pieces (51a) of the stop rim (51) are widened outwards by the protruding step (41c) so that the push button (52) and the piston (41), which are adhered to each other, are pushed downwards to discharge the fixed quantity of liquid to the outside of the cylinder (40) only when the fixed quantity of liquid is charged in the cylinder (40); and
said the continuous injection unit (64) comprises:
a connector (60) that has an inward recess (60a), one side of which is mounted on the discharge hole (40b) of the cylinder (40) and an inward hole that passes through the inward recess (60a) laterally;
a connection pipe (61) mounted on an outlet port (32) of the cases; and
a resilient tube (62) fixedly installed between the connector (60) and the connection pipe (61),
wherein the fixed quantity of liquid discharged to the outside of the discharge hole (40b) of the cylinder (40) is accommodated in the interior of the resilient tube (62) through the inward hole (60b), and
wherein a small quantity of liquid is continuously sent to the outside of the connection pipe (61) by a force by which the resilient tube (62) contracted while accommodating the fixed quantity of liquid discharged to the outside of the cylinder (40) is contracted.

2. The liquid injector (30) of claim 1,
wherein a pair of coupling pieces (60c) that are cut away longitudinally and face each other are formed on an opposite side of the connector (60) and coupling steps (60d) are formed at ends of the coupling pieces (60c), and
wherein the coupling pieces (60c) are inserted into the connection pipe (61) to be stopped and fixed by coupling recess (61a) so that the liquid accommodated in the interior of the resilient tube (62) is injected into the body of the patient while being discharged to the outside of the connection pipe (61) through a cutaway portion (60e) between the pair of coupling pieces (60c) when the resilient tube (62) is contracted and expanded with respect to the coupling pieces (60c).

3. The liquid injector (30) of any of claims 1 or 2,
wherein a fixed tube (63) is installed inside the resilient tube (62), and
wherein the fixed tube (63) is fixedly installed while surrounding the inward hole of the connector (60) so that the liquid discharged to the outside of the inward hole is discharged to the outside of the fixed tube (63) and is accommodated only in the interior of the resilient tube (62).

4. The liquid injector (30) of claim 1, wherein lower ends of the stop pieces (51a) are cutaway such that the stop pieces (51a) located on the upper side are easily resiliently supported by the stop rim (51).

5. The liquid injector (30) of claim 1, wherein
the brake button unit (53) has a stop groove (55) having a fixing boss (55a) formed long inside the case up to a lower portion of an upper end periphery of the case (30a, 30b),
a recess (56a) corresponding to the fixing boss (55) is formed at one side of the stop groove (55) and a lock spring (56) having an inclined piece (55b) is mounted at an opposite side thereof in an inward direction,
an end of the inclined piece (55b) is located between a support step (52a) of the push button (52) located on the upper side and a protruding step (41c) of the piston (41),
the push button (52) is stopped by the inclined piece (55b) of the lock spring (56) such that a downward movement of the push button (52) is braked in a normal state, and
the piston (41) is moved downwards together with the push button (52) so that a fixed amount of liquid filled in the cylinder (40) may be discharged to the outside only when a fixed amount of liquid is filled in the interior of the cylinder (40) as the piston (41) ascends to the end such that the inclined piece (55b) of the lock spring (56) is pushed to the outside by the protruding step (41c) and the support end (52a) of the push button (52) and the protruding step (41c) of the piston (41) make contact with each other.

6. The liquid injector (30) of claim 1 or 5, wherein a transparent window is formed on one side of the case (30a, 30b) of the liquid injector (30) and a cylinder (40) in the case (30a, 30b) is also formed of a transparent material so that a liquid can be visually identified through the transparent window if the cylinder (40) is filled with the liquid

7. The liquid injector (30) of claim 1 or 5, wherein
a mounting recess (57) is formed inside an upper end periphery of the case (30a, 30b), and
the key (70) including a knob (71) protruding to the upper side of the push button (52), a mounting boss (72) that allows the support step (52a) of the push button (52) to be located inside the stop piece (51b) or the inclined piece (56b) of the lock spring (56) when being inserted into the mounting recess (57), and a pair of resilient support pieces formed at a lower portion of the mounting boss (72) is detachably installed in the mounting recess (57).

## Patentansprüche

1. Flüssigkeitsinjektor (30), der geladene Flüssigkeit zusätzlich zuführt, während er kontinuierlich eine geringe Menge an Flüssigkeit injiziert, wobei der Flüssigkeitsinjektor (30) folgendes umfasst:
eine Fixmengen-Ladeeinheit (43), die eine feste Menge an Flüssigkeit lädt, eine Bremsknopfeinheit (53), die die in der Fixmengen-Ladeeinheit (43) geladene Flüssigkeit entlädt, und eine Einheit für kontinuierliche Einspritzung (64), die die Flüssigkeit nach und nach kontinuierlich zu einem Injektorschlauch (20) sendet, während sie die durch die Bremsknopfeinheit (53) entladene Flüssigkeit so unterbringt, dass die Flüssigkeit nur dann entladen wird, wenn eine feste Menge an Flüssigkeit in der Fixmengen-Ladeeinheit (43) geladen ist, und die entladene Flüssigkeit dann nach und nach kontinuierlich in den Körper des Patienten injiziert wird, wobei
die Fixmengen-Ladeeinheit (43) an einem unteren Ende eines Druccknopfes (52) angehaftet ist, wenn ein Kolben durch in das Innere eines Zylinders eingeführte Flüssigkeit angehoben und dann gestoppt wird, um eine feste Menge an Flüssigkeit im Inneren des Zylinders zu laden;
die Bremsknopfeinheit (53) dann, wenn der angehobene Kolben und der Druckknopf (52) aneinander angehaftet sind, während die Abwärtsbewegung des Druckknopfes (52) gestoppt wird, den Druckknopf (52) zusammen mit dem Kolben nach unten absenkt, da die gestoppte Abwärtsbewegung des Druckknopfes (52) freigegeben wird; und
die Einheit für kontinuierliche Einspritzung (64) durch eine Rückstellkraft eines elastischen Rohrs (62), das während eines Unterbringens einer durch die Bremsknopfeinheit (53) zur Außenseite des Zylinders entladenen festen Menge an Flüssigkeit ausgedehnt wird, die im Inneren des elastischen Rohrs (62) untergebrachte Flüssigkeit kontinuierlich nach außen drückt,
**dadurch gekennzeichnet, dass**
die Fixmengen-Ladeeinheit (43) folgendes umfasst:
einen Zylinder (40), der auf einer Seite davon ein Ladeloch und ein Entladeloch hat und der auf einer gegenüberliegenden Seite davon einen Unterbringungsraum hat;
einen Kolben (41), dessen unteres Ende in den Unterbringungsraum des Zylinders (40) eingefügt ist, der an einem Ende davon einen Einfügeraum (41b) hat und der an einem Umfang davon eine vorstehende Stufe (41c) hat;
einen elastischen Körper, der in den Einfügeraum (41b) des Kolbens (41) eingefügt ist; und
einen Druckknopf (52), der an einem Umfang davon über dem elastischen Körper eine Stützstufe (52a) hat, und
wobei die vorstehende Stufe (41c) und die Stützstufe (52a) des Druccknopfes (52) aneinander angehaftet sind, wenn ein Kolben (41) durch die in das Innere des Zylinders (40) eingeführte Flüssigkeit angehoben wird, und dann an einer vorbestimmten Stelle gestoppt wird, um eine feste Menge an Flüssigkeit im Inneren des Zylinders (40) zu laden;
die Bremsknopfeinheit (53) folgendes umfasst:
einen ringförmigen Stopprand (51), der nach innen gerichtete Stoppstücke (51a) hat; und
Gehäuse (30a, 30b), von denen jedes eine Stoppnut (50) hat, in der der Stopprand (51) positioniert ist, und eine Führungsstange (54) hat,
wobei der Kolben (41) auf der Innenseite der Gehäuse (30a, 30b) montiert ist, während er in den Stopprand (51) eingefügt ist, und
wobei dann, wenn der Kolben (41) angehoben ist, eine Stützstufe (52a) des Druckknopfes (52), die an den Stoppstücken (51a) montiert ist, und die vorstehende Stufe (41c) des Kolbens (41) aneinander angeheftet sind, während die Stoppstücke (51a) des Stopprands (51) durch die vorstehende Stufe (41c) so nach außen aufgeweitet sind, dass der Druckknopf (52) und der Kolben (41), die aneinander angeheftet sind, nur nach unten gedrückt werden, um die feste Menge an Flüssigkeit zur Außenseite des Zylinders (40) zu entladen, wenn die feste Menge an Flüssigkeit im Zylinder (40) geladen ist; und
die Einheit für kontinuierliche Einspritzung (64) folgendes umfasst:
ein Anschlussstück (60), das eine nach innen gerichtete Aussparung (60a) hat, von dem eine Seite an dem Entladeloch (40b) des Zylinders (40) und einem nach innen gerichteten Loch, das lateral durch die nach innen gerichtete Aussparung (60a) verläuft, montiert ist;
ein Anschlussrohr (61), das an einem Auslasstor (32) der Gehäuse montiert ist; und
ein elastisches Rohr (62), das zwischen dem Anschlussstück (60) und dem Anschlussrohr (61) fest installiert ist,
wobei die zur Außenseite des Entladelochs (40b) des Zylinders (40) entladene feste Menge an Flüssigkeit durch das nach innen gerichtete Loch (60b) im Inneren des elastischen Rohrs (62) untergebracht wird, und
wobei eine geringe Menge an Flüssigkeit durch eine Kraft, durch die das während eines Unterbringens der zur Außenseite des Zylinders (40) entladenen festen Menge an Flüssigkeit zusammengezogene elastische Rohr (62) zusammengezogen wird, kontinuierlich zur Außenseite des Anschlussrohrs (61) gesendet wird.

2. Flüssigkeitsinjektor (30) nach Anspruch 1,
wobei ein Paar von Kopplungsstücken (60c), die in Längsrichtung abgeschnitten sind und einander zugewandt sind, auf einer gegenüberliegenden Seite des Anschlussstücks (60) ausgebildet ist und an Enden der Kopplungsstücke (60c) Kopplungsstufen (60d) ausgebildet sind, und
wobei die Kopplungsstücke (60c) in das Anschlussrohr (61) eingefügt sind, um durch einen Kopplungsausschnitt (61a) gestoppt und fixiert zu werden, so dass die im Inneren des elastischen Rohrs (62) untergebrachte Flüssigkeit in den Körper des Patienten injiziert wird, während sie zur Außenseite des Anschlussrohrs (61) durch einen abgeschnittenen Teilbereich (60e) zwischen dem Paar von Kopplungsstücken (60c) entladen wird, wenn das elastische Rohr (62) in Bezug auf die Kopplungsstücke (60c) zusammengezogen und ausgedehnt wird.

3. Flüssigkeitsinjektor (30) nach einem der Ansprüche 1 oder 2,
wobei ein festes Rohr (63) innerhalb des elastischen Rohrs (62) installiert ist, und
wobei das feste Rohr (63) fest installiert ist, während es das nach innen gerichtete Loch des Anschlussstücks (60) umgibt, so dass die zur Au-ßenseite des nach innen gerichteten Lochs entladene Flüssigkeit zur Außenseite des festen Rohrs (63) entladen wird und nur im Inneren des elastischen Rohrs (62) untergebracht ist.

4. Flüssigkeitsinjektor (30) nach Anspruch 1, wobei die unteren Enden der Stoppstücke (51a) so abgeschnitten sind, dass die auf der oberen Seite angeordneten Stoppstücke (51a) durch den Stopprand (51) auf einfache Weise elastisch gestützt sind.

5. Flüssigkeitsinjektor (30) nach Anspruch 1, wobei
die Bremsknopfeinheit (53) eine Stoppnut (55) mit einem lang innerhalb des Gehäuses bis zu einem unteren Teilbereich einer oberen Endperipherie des Gehäuses (30a, 30b) ausgebildeten runden Fixiervorsprung (55a) hat,
eine dem runden Fixiervorsprung (55) entsprechende Aussparung (56a) auf einer Seite der Stoppnut (55) ausgebildet ist und eine Verriegelungsfeder (56) mit einem geneigten Stück (55b) auf einer gegenüberliegenden Seite davon in einer nach innen gerichteten Richtung montiert ist,
ein Ende des geneigten Stücks (55b) zwischen einer Stützstufe (52a) des auf der oberen Seite angeordneten Druckknopfes (52) und einer vorstehenden Stufe (41c) des Kolbens (41) angeordnet ist,
der Druckknopf (52) durch das geneigte Stück (55b) der Verriegelungsfeder (56) so gestoppt wird, dass eine Abwärtsbewegung des Druccknopfes (52) in einem normalen Zustand gebremst wird, und
der Kolben (41) zusammen mit dem Druckknopf (52) nach unten bewegt wird, so dass eine in den Zylinder (40) gefüllte feste Menge an Flüssigkeit nur dann zur Außenseite entladen werden kann, wenn eine feste Menge an Flüssigkeit in das Innere des Zylinders (40) gefüllt ist, da der Kolben (41) zum Ende aufsteigt, so dass das geneigte Stück (55b) der Verriegelungsfeder (56) durch die vorstehende Stufe (41c) nach außen gedrückt wird und das Stützende (52a) des Druckknopfes (52) und die vorstehende Stufe (41c) des Kolbens (41) einen Kontakt zueinander herstellen.

6. Flüssigkeitsinjektor (30) nach Anspruch 1 oder 5, wobei ein transparentes Fenster auf einer Seite des Gehäuses (30a, 30b) des Flüssigkeitsinjektors (30) ausgebildet ist und ein Zylinder (40) im Gehäuse (30a, 30b) ebenfalls aus einem transparenten Material ausgebildet ist, so dass eine Flüssigkeit durch das transparente Fenster hindurch visuell identifiziert werden kann, wenn der Zylinder (40) mit der Flüssigkeit gefüllt ist.

7. Flüssigkeitsinjektor (30) nach Anspruch 1 oder 5, wobei
ein Montageausschnitt (57) innerhalb einer oberen Endperipherie des Gehäuses (30a, 30b) ausgebildet ist, und
der Schlüssel (70), der einen zur oberen Seite des Druckknopfes (52) vorstehenden Handgriff (71) enthält, einen runden Montagevorsprung (72), der zulässt, dass die Stützstufe (52a) des Druckknopfes (52) innerhalb des Stoppstücks (51b) oder des geneigten Stücks (56b) der Verriegelungsfeder (56) angeordnet ist, wenn sie in den Montageausschnitt (57) eingefügt ist, und ein Paar von elastischen Stützstücken, die an einem unteren Teilbereich des Montagevorsprungs (72) ausgebildet sind, herausnehmbar im Montageausschnitt (57) installiert ist.

## Revendications

1. Injecteur de liquide (30) fournissant en plus un liquide chargé tout en injectant en continu une petite quantité de liquide, l'injecteur de liquide (30) comprenant :
une unité de chargement de quantité fixe (43) qui charge une quantité fixe de liquide, une unité de bouton de freinage (53) qui décharge le liquide chargé dans l'unité de chargement de quantité fixe (43), et une unité d'injection continue (64) qui envoie en continu le liquide à un tuyau d'injecteur (20) petit à petit tout en recevant le liquide déchargé par l'unité de bouton de freinage (53) de sorte que le liquide est déchargé uniquement lorsqu'une quantité fixe de liquide est chargée dans l'unité de chargement de quantité fixe (43) et ensuite le liquide déchargé est injecté en continu dans le corps du patient petit à petit, dans lequel
ladite unité de chargement de quantité fixe (43) est collée à une extrémité inférieure d'un bouton-poussoir (52) lorsqu'un piston est soulevé par du liquide introduit à l'intérieur d'un cylindre et est ensuite arrêté pour charger une quantité fixe de liquide à l'intérieur du cylindre ;
ladite unité de bouton de freinage (53), si le piston soulevé et le bouton-poussoir (52) sont collés l'un à l'autre alors que le mouvement descendant du bouton-poussoir (52) est arrêté, abaisse le bouton-poussoir (52) en même temps que le piston vers le bas lorsque le mouvement descendant arrêté du bouton-poussoir (52) est libéré ; et
ladite unité d'injection continue (64), par une force de rappel d'un tube élastique (62) qui est dilaté tout en recevant une quantité fixe de liquide déchargée à l'extérieur du cylindre par l'unité de bouton de freinage (53), pousse continuellement le liquide logé à l'intérieur du tube élastique (62),
**caractérisé par le fait que**
ladite unité de chargement à quantité fixe (43) comprend :
un cylindre (40) comportant un orifice de chargement et un orifice de déchargement sur un de ses côtés et un espace de logement sur le côté opposé ;
un piston (41) dont l'extrémité inférieure est insérée dans l'espace de logement du cylindre (40), comportant un espace d'insertion (41b) à une extrémité de celui-ci, et un épaulement en saillie (41c) à la périphérie de celui-ci ;
un corps élastique, inséré dans l'espace d'insertion (41b) du piston (41) ; et
un bouton-poussoir (52) comportant une saillie de support (52a) à sa périphérie au-dessus du corps élastique, et
dans lequel l'épaulement en saillie (41c) et la saillie de support (52a) du bouton poussoir (52) sont collés l'un à l'autre lorsqu'un piston (41) est soulevé par le liquide introduit à l'intérieur du cylindre (40), puis est arrêté à un emplacement prédéterminé pour charger une quantité fixe de liquide à l'intérieur du cylindre (40) ;
ladite unité de bouton de freinage (53) comprend :
un rebord d'arrêt de forme annulaire (51) doté de pièces d'arrêt vers l'intérieur (51a) ; et des boîtiers (30a, 30b) chacun équipé d'une rainure d'arrêt (50) dans laquelle le rebord d'arrêt (51) est positionné et possède une tige de guidage (54).
dans lequel le piston (41) est monté sur l'intérieur des boîtiers (30a, 30b) tout en étant inséré dans le rebord d'arrêt (51), et
dans lequel, si le piston (41) est soulevé, la saillie de support (52a) du bouton-poussoir (52) monté sur les pièces d'arrêt (51a) et l'épaulement en saillie (41c) du piston (41) sont collés l'un à l'autre tandis que les pièces d'arrêt (51a) du rebord d'arrêt (51) sont élargies vers l'extérieur par l'épaulement en saillie (41c) de sorte que le bouton-poussoir (52) et le piston (41), qui sont collés l'un à l'autre, sont poussés vers le bas pour décharger la quantité fixe de liquide vers l'extérieur du cylindre (40) uniquement lorsque la quantité fixe de liquide est chargée dans le cylindre (40) ; et
ladite unité d'injection continue (64) comprend :
un connecteur (60) comportant un évidement intérieur (60a), dont un côté est monté sur l'orifice de décharge (40b) du cylindre (40) et un orifice intérieur qui traverse latéralement l'évidement intérieur (60a) ;
un tuyau de connexion (61) monté sur un orifice de sortie (32) des boîtiers ; et
un tube élastique (62) installé de manière fixe entre le connecteur (60) et le tuyau de connexion (61),
dans lequel la quantité fixe de liquide déchargée vers l'extérieur de l'orifice de décharge (40b) du cylindre (40) est logée à l'intérieur du tube élastique (62) à travers le l'orifice d'entrée (60b), et
dans lequel une petite quantité de liquide est envoyée en continu vers l'extérieur du tuyau de connexion (61) par une force par laquelle le tube élastique (62) contracté en recevant la quantité fixe de liquide déchargée vers l'extérieur du cylindre (40) est contracté.

2. Injecteur de liquide (30) selon la revendication 1,
dans lequel une paire de pièces d'accouplement (60c) qui sont découpées longitudinalement et se font face l'une à l'autre sont formées sur un côté opposé du connecteur (60) et des crans d'accouplement (60d) sont formés aux extrémités des pièces d'accouplement (60c), et
dans lequel les pièces d'accouplement (60c) sont insérées dans le tuyau de connexion (61) pour être arrêtées et fixées par un creux d'accouplement (61a) de sorte que le liquide logé à l'intérieur du tube élastique (62) est injecté dans le corps du patient tout en étant évacué vers l'extérieur du tuyau de connexion (61) à travers une partie découpée (60e) entre la paire de pièces d'accouplement (60c) lorsque le tube élastique (62) est contracté et dilaté par rapport aux pièces d'accouplement (60c).

3. Injecteur de liquide (30) selon l'une quelconque des revendications 1 ou 2,
dans lequel un tube fixe (63) est installé à l'intérieur du tube élastique (62), et
dans lequel le tube fixe (63) est installé de manière fixe tout en entourant l'orifice intérieur du connecteur (60) de sorte que le liquide déchargé vers l'extérieur de l'orifice intérieur est déchargé vers l'extérieur du tube fixe (63) et est logé uniquement à l'intérieur du tube élastique (62).

4. Injecteur de liquide (30) selon la revendication 1, dans lequel les extrémités inférieures des pièces d'arrêt (51a) sont découpées de telle sorte que les pièces d'arrêt (51a) situées sur le côté supérieur sont facilement supportées de manière élastique par le rebord d'arrêt (51).

5. Injecteur de liquide (30) selon la revendication 1, dans lequel
l'unité de bouton de freinage (53) comprend une rainure d'arrêt (55) ayant un bossage de fixation (55a) formé le long de l'intérieur du boîtier jusqu'à une partie inférieure d'une périphérie d'extrémité supérieure du boîtier (30a, 30b),
un évidement (56a) correspondant au bossage de fixation (55) est formé sur un côté de la rainure d'arrêt (55) et un ressort de blocage (56) doté d'une pièce inclinée (55b) est monté sur le côté opposé de celui-ci en direction de l'intérieur,
une extrémité de la pièce inclinée (55b) est située entre la saillie de support (52a) du bouton-poussoir (52) située sur le côté supérieur et l'épaulement en saillie (41c) du piston (41),
le bouton-poussoir (52) est arrêté par la pièce inclinée (55b) du ressort de verrouillage (56) de sorte qu'un mouvement vers le bas du bouton-poussoir (52) est freiné dans un état normal, et
le piston (41) est déplacé vers le bas en même temps que le bouton-poussoir (52), de sorte qu'une quantité fixe de liquide remplissant le cylindre (40) peut être déchargée vers l'extérieur uniquement lorsqu'une quantité fixe de liquide est remplie à l'intérieur du cylindre (40) lorsque le piston (41) monte vers l'extrémité de sorte que la pièce inclinée (55b) du ressort de verrouillage (56) est poussée vers l'extérieur par l'épaulement en saillie (41c) et l'extrémité de support (52a) du bouton-poussoir (52) et l'épaulement en saillie (41c) du piston (41) entrent en contact l'un avec l'autre.

6. Injecteur de liquide (30) selon la revendication 1 ou 5, dans lequel une fenêtre transparente est formée sur un côté du boitier (30a, 30b) de l'injecteur de liquide (30) et un cylindre (40) dans le boitier (30a, 30b) est également formé à partir d'un matériau transparent de sorte qu'un liquide peut être visuellement identifié à travers la fenêtre transparente si le cylindre (40) est rempli du liquide.

7. Injecteur de liquide (30) selon la revendication 1 ou 5, dans lequel
un évidement de montage (57) est formé à l'intérieur de la périphérie de l'extrémité supérieure du boîtier (30a, 30b), et
la clé (70) comprenant un bouton (71) faisant saillie sur le côté supérieur du bouton-poussoir (52), un bossage de montage (72) qui permet à la saillie de support (52a) du bouton-poussoir (52) d'être située à l'intérieur de la pièce d'arrêt (51b) ou de la pièce inclinée (56b) du ressort de verrouillage (56) lorsqu'elle est insérée dans l'évidement de montage (57), et une paire de pièces de support élastiques en éventail au niveau de la partie inférieure du bossage de montage (72) est installée de manière amovible dans l'évidement de montage (57).
